# EUROPEAN PATENT APPLICATION

(11) **EP 1 365 026 A1**
(43) Date of publication of application: **26.11.2003**
(21) Application number: 02705050.9
(22) Date of filing: 27.02.2002
(51) Int. Cl.: C12N 15/61, C12Q 1/68, C12N 1/15, C12N 1/19, C12N 1/21, C12N 5/10, C12R 1/21

(54) **DNA ENCODING L-RIBOSE ISOMERAE AND USE THEREOF**

(30) Priority: 01.03.2001 JP 2001057416
(71) Applicant: KABUSHIKI KAISHA HAYASHIBARA SEIBUTSU KAGAKU KENKYUJO, Okayama-shi Okayama 700-0907 (JP)
(72) Inventor: IZUMORI, Ken, Takamatsu-shi, Kagawa 761-8078 (JP); TSUSAKI, Keiji, Hayashibara Seibutsu Kag. Ken. KK., Okayama-shi, Okayama 700-0907 (JP)
(74) Representative: Daniels, Jeffrey Nicholas
(86) International application number: JP0201809
(87) International publication number: WO02070718

(57) **Abstract**

The present invention solves the object of the present invention by providing a DNA encoding an L-ribose isomerase which isomerizes L-ribose into L-ribulose and *vice versa,* and the process for producing a polypeptide by recombinant DNA techniques using the DNA.

## Description

### TECHNICAL FIELD

The present invention relates to a novel DNA, more particularly, to a DNA encoding an L-ribose isomerase and uses thereof.

### BACKGROUND ART

L-Ribose is a type of rare sugars whose industrial productions have not been established. Recently, the usefulness of this saccharide has been highlighted in the fields of foods and pharmaceuticals as materials for producing anti-viral agents and anti-cancer agents. Therefore, it has been strongly required to establish the industrial-scale production of the saccharide.

To attain the requirement, the present inventors disclosed a novel enzyme, L-ribose isomerase which is useful for the industrial-scale production of L-ribose, the process for producing the enzyme using a microorganism capable of producing the enzyme, and its uses in Japanese Patent Kokai No. 155,480/98, applied for by the same applicant as the present invention.

As described above, the industrial-scale production of L-ribose has established as the research result by the present inventors. In the course of further studies on L-ribose isomerase, however, the present inventors found that further improvement in production efficiency of the enzyme and in enzymatic properties is needed, and that there has been no study to overcome such requirement.

Under these circumstances, if a DNA encoding an L-ribose-forming enzyme will be cloned, application of the recombinant DNA technique, which has been in a remarkable progress in these days, to such a DNA would meet the above requirement. However, there is no positive report on the cloning of a DNA encoding an L-ribose-forming enzyme.

### DISCLOSURE OF INVENTION

To attain these objects, the present inventors extensively screened chromosomal DNAs from L-ribose isomerase-producing microorganisms by using the partial amino acid sequences disclosed in Japanese Patent Kokai No. 155,480/98, applied for by the same applicant as the present invention. As a result, they successfully cloned a DNA encoding an amino acid sequence which has the partial amino acid sequence described above, from a microorganism of the genus *Acinetobacter.* They also found that recombinant microorganisms having the cloned DNA efficiently produced a polypeptide having L-ribose isomerase activity. Thus, the present invention was accomplished based on the research results by the present inventors.

The present invention solves the first object by providing a DNA encoding an L-ribose isomerase which catalyzes the isomerization reaction of L-ribose into L-ribulose and *vice versa* (hereinafter, the term "L-ribose isomerase activity" means the enzyme activity which catalyzes these isomerization reactions).

In addition, the present invention solves the second object by providing the uses of the DNA, particularly, by providing a process comprising the steps of artificially expressing the DNA to produce a polypeptide having L-ribose isomerase activity, and collecting the produced polypeptide.

### BEST MODE FOR CARRYING OUT THE INVENTION

The present invention relates to a DNA encoding an L-ribose isomerase and its uses. The L-ribose isomerase as referred to in the present invention means an enzyme having L-ribose isomerase activity or an enzyme that converts L-ribose into L-ribulose and *vice versa.* The L-ribose isomerase activity can be detected by the following assay: The reaction mixture is prepared by mixing 0.05 ml of 0.5 M glycine-sodium hydroxide buffer (pH 9.0), 0.05 ml of 0.05 M L-ribose, and 0.4 ml of an enzyme solution. The resulting mixture is incubated at 30°C for 10 minutes to isomerize L-ribose into L-ribulose. After stopping the reaction by boiling, the amount of L-ribulose formed in the reaction mixture is determined by the cysteine-carbazole method. In the present invention, one unit of L-ribose isomerase activity is defined as the amount of enzyme that forms one micromole of L-ribulose per minute under the above conditions.

Concrete examples of the L-ribose isomerase according to the present invention include those which have the whole of the amino acid sequence of SEQ ID NO:1. These L-ribose isomerases having the amino acid sequence can be isolated from microorganisms, particularly those of the genus *Acinetobacter* capable of producing the enzyme. L-Ribose isomerases are usually isolated from such microorganisms as oligomeric enzymes constructed by four subunits of a polypeptide having the amino acid sequence of SEQ ID NO:1. The amino acid sequence in SEQ ID NO:1 is an example of the amino acid sequence which is contained in the L-ribose isomerase according to the present invention. The L-ribose isomerase may comprise an amino acid sequence which is not fully identical with SEQ ID NO: 1, for example, those which methionine is added to the N-terminus of SEQ ID NO:1, or in which one or more amino acid residues at the N- and/or C-termini of SEQ ID NO:1 are deleted. Thus, the L-ribose isomerase according to the present invention is not restricted to the enzyme or polypeptide having the amino acid sequence of SEQ ID NO:1, and includes those which exhibit L-ribose isomerase activity and have a partial amino acid sequence of SEQ ID NO:1. L-Ribose isomerases having partial amino acid sequence of SEQ ID NO:1 have, usually, a homology of 60% or higher, preferably, 70% or higher, more preferably, 80% or higher, and most preferably, 90% or higher when calculated by conventional method based on maximum-matching alignment between two amino acid sequences.

The DNAs of the present invention encode the L-ribose isomerase as defined above. They comprises any one of those in the form of a single or double strand nucleotide chain as long as they encode the L-ribose isomerase (hereinafter, a chain constructed by the nucleotide sequence which encodes the L-ribose isomerase may be called " a sense chain"). For the concrete examples of the DNA of the present invention, those which contain the whole of the nucleotide sequence of SEQ ID NO:2 that encodes the amino acid sequence of SEQ ID NO:1 are examplified. DNAs encoding for the above amino acid sequences can be, generally, isolated from microorganisms which produce the enzyme; Particularly, the DNAs can be isolated from the chromosomal DNAs of microorganisms of the genus *Acinetobacter.* Generally, these isolated DNAs contain the nucleotide sequence of SEQ ID NO: 2 which has an initial and a stop codon added to the 5 ' - and 3'-termini, respectively. The nucleotide sequence of SEQ ID NO:2 is an example of the DNA of the present invention. The DNAs of the present invention may contain other nucleotide sequences which do not completely correspond to the nucleotide sequence of SEQ ID NO:2. In general, the DNAs of the present invention can be modified by replacement, insertion and deletion of nucleotides by applying conventional recombinant DNA techniques such as site-directed mutagenesis to DNAs with specific nucleotides. Such modification include the addition of nucleotide sequences, which encode (His)₆-tag having affinity for specific materials, or which encode signal peptides. Therefore, the DNAs according to the present invention are not restricted to those which comprise a part or the whole of the nucleotide sequence of SEQ ID NO: 2, and which encode the desired L-ribose isomerase. Compared with the nucleotide sequence of SEQ ID NO;2, the DNAs of the present invention having partial nucleotide sequence of SEQ ID NO:2 have, usually, a homology of 60% or higher, preferably, 70% or higher, more preferably, 80% or higher, and most preferably, 90% or higher when calculated by conventional method based on maximum-matching alignment.

The DNAs of the present invention are those defined in the above and are not restricted to those which are obtained by specific preparation methods. The DNAs of the present invention can be obtained, for example, by screening based on either the nucleotide sequence disclosed in the specification, or the amino acid sequence of L-ribose isomerase that is encoded by a DNA from chromosomal DNAs of microorganisms of the genus *Acinetobacter,* more particularly, *Acinetobacter calcoaceticus* LR7C (FERM BP-5335), and mutants thereof, gram-positive bacteria, gram-negative bacteria, yeasts, fungi, and other microorganisms by using conventional gene cloning techniques. To screen chromosomal DNAs, hybridization method of genomic library, PCR method using chromosomal DNAs as templates, and modified methods thereof are applicable. To practice the above screening, the following DNAs are advantageously used: The DNAs of the present invention including those in a single- or double-stranded forms and in single-stranded DNAs constructed by an anti-sense DNA corresponding to the sense chains of the DNAs of the present invention, and include fragments of these DNAs having usually at least 10 contiguous bases, preferably, at least 12 contiguous bases, more preferably, at least 15 contiguous bases, more preferably, at least 20 contiguous bases. On the screening from genomic library, for example, the DNAs of the present invention are obtainable by a method which comprises the steps of labeling the DNAs or the fragments described above with radioisotopes, enzymes or digoxigenin; operating a hybridization on the genomic library using the labeled DNAs as hybridization probes; and collecting a DNA from a genomic clone showing remarkable hybridization with the probe. To practice the PCR-method, the DNAs of the present invention can be obtained from PCR-amplified DNAs by using oligo-nucleotide having the DNA fragments described above as a PCR-primer (sense primer and/or anti-sense primer). If necessary, these methods can be appropriately applied in combination. The DNAs of the present invention can also be obtained by usual chemical synthetic methods according to the nucleotide sequence of DNAs disclosed in the present invention.

The DNAs of the present invention can be advantageously used to produce polypeptides, more particularly, those having L-ribose isomerase activity, by recombinant DNA technique, and the present invention provides the production method thereof. The process for producing the polypeptide according to the present invention is characterized in that it comprises the steps of expressing artificially the DNA of the present invention to produce a polypeptide having L-ribose isomerase activity, and isolating the polypeptide. In order to express the DNAs artificially, for example, it is possible to use a general *in vitro* expression method including *in vitro* transcription and translation methods. To produce the desired polypeptide on an industrial scale, it is preferable to cultivate the transformed host cells prepared by transforming with the DNA of the present invention.

Usually, transformants used in the present process can be obtained by transforming appropriate hosts with the recombinant DNAs constructed by inserting the DNAs of the present invention into autonomously replicable vectors. Depending on the types of hosts used, autonomously replicable vectors can be appropriately selected from conventional vectors, for instance, plasmid vectors such as pBR322, pUC18, Bluescript II SK(+), pUB110, pTZ4, pC194, pHV14, TRp7, Yep7 and pBS7; or phage vectors such as λgt · λC, λgt · λB, ρ11, φ1 and φ105. To express the DNAs of the present invention in *E. coli,* pUC118, pUC119, pUC18, pUC19, pBR322, Bluescript II SK(+), λgt · λC and λgt · λB are preferably used among the vectors described above. To express the DNAs of the present invention in *B. subtilis,* pUB110, pTZ4, pC194, ρ11, φ1 and φ105 are useful. pHV14, TRp7, YEp7 and pBS7 are useful to express the desired recombinant DNAs in two or more hosts. These autonomously replicable vectors generally contain nucleotide sequences such as promoters, enhancers, replication origins, transcriptional terminators, and selection sequences to express the present DNAs in various hosts, or to confirm the desired transformation. In order to insert the DNAs of the present invention into these vectors, conventional methods used in the art can be arbitrarily used. For example, addition of linkers, addition of restriction enzyme sites synthesized by PCR-method, restriction enzyme treatment, and ligase treatment can be used.

Host cells conventionally used for the construction of transformants, for instance, microorganisms such as *E. coli, B. subtilis*, yeast, and fungi, invertebrates such as insects, plants, and vertebrates, can be used as the host cells into which the DNAs of the present invention are introduced. To provide the polypeptide at a low cost, it is preferable to use microorganisms such as *E. coli* and *B. subtilis* as the host cells. In order to transform the DNAs of the present invention into the host cells, for example, calcium phosphate method, electroporation method, and virus-transfection method, if necessary, DEAE-dextran method, lipo-fection method and micro-injection method can be arbitrarily used. The desired clones can be selected from the transformants with a maker of the transformed-DNA or the polypeptide productivity is useful. Details of common materials and methods for recombinant DNA techniques and transformants are described by J. Sambrook *et al.* in the 2nd Edition of *molecular Cloning, a laboratory manual* (Cold Spring Harbor Lab. 1989).

The transformed cells thus obtained produce the polypeptides having L-ribose isomerase activity extra/intra-cellularly when cultivated under conditions of an appropriate inductive stimulation, if necessary; depending on the kind of the host cells or vectors used in introducing the desired DNAs. Varying depending on the kind of the host cells and vectors used, preferably used are culture media used in general which are supplimented with carbon sources, nitrogen sources and minerals, furthermore, if necessary, with trace-nutrients such as amino acids and vitamins. Examples of the carbon sources usable in the present invention include starches, starch hydrolyzates, glucose, fructose, sucrose and trehalose. Examples of the nitrogen sources usable in the present invention are nitrogen containing inorganic- or organic-substances including ammonia, ammonium salts, urea, nitrate, peptone, yeast extract, defatted soybean, corn-steep liquor and meat extract. Cultures containing the polypeptides having L-ribose isomerase activity can be obtained when the transformed cells are cultivated for one to five days under aerobic conditions such as aeration and agitation conditions while keeping the temperature and pH, usually, at 20-60°C, and pH 2-10 which are varied depending on the host cells and vectors used.

Although the polypeptides produced in the above process can be used intact, if necessary, they can be purified before use. The following purification procedures for proteins commonly used in the art can be used: salting out, dialysis, filtration, concentration, precipitation, ion-exchange chromatography, gel filtration chromatography, adsorption chromatography, isoelectrofocusing chromatography, hydrophobic chromatography, reverse-phase chromatography, affinity chromatography, gel electrophoresis, and isoelecric focusing. The desired polypeptides purified to the desired level can be obtained by analyzing properties such as amino acid sequence, molecular weight and isomerase activity to determine the desired fractions and collecting the fractions which show the desired properties.

Similarly as the L-ribose isomerase disclosed in Japanese Patent Kokai No. 155,480/98 applied for by the same applicant as the present invention, the polypeptides having L-ribose isomerase activty thus obtained have such an activity and other activities that catalyze the isomerization reaction of aldoses such as D-lyxose, D-talose, D-mannose, L-allose and L-gulose. The polypeptides obtained by the process according to the present invention can be advantageously supplied at a relatively lower cost than those for natural enzymes or for enzymes produced from microorganisms capable of producing L-ribose isomerase. The above polypeptides are very useful for the industrial production of saccharides such as L-ribose and D-talose which have been useful but rare.

The DNAs of the present invention, anti-sense single stranded DNAs and fragments thereof are very useful for the screening of other enzymes having similar structures to that of L-ribose isomerase. It is possible that DNAs encoding a novel enzyme having a different activity from L-ribose isomerase activity can be obtained by the screening of chromosomal DNAs prepared from various origins according to the methods of hybridization or PCR used to prepare the DNAs of the present invention. Conditions (stringencies) for hybridization of probes and chromosomal DNAs or for annealing of PCR primers and chromosomal DNAs can be advantageously used. Stringency for hybridization or annealing is generally influenced by temperature and ionic strength. Details of factors which influence on the determination of stringency are described by J. Sambrook *et al.* in the 2nd Edition of *Molecular Cloning, a laboratory-manual* (Cold Spring Harbor Lab. 1989). With reference to the details, various DNAs encoding enzymes which show different structural similarities to L-ribose isomerase can be obtained by hybridization or PCR-method under various stringencies. It is possible that novel enzymes which are usable to produce rare sugars, as variations of the present invention, can be obtained by the expression of the above DNAs using convenient recombinant DNA techniques and by the assay for enzyme activity of the expression products. In addition, it is possible to attain the molecular design for the purpose of changing the substrate specificity and enzymatic properties by combining the knowledge for the structure-function relationship between L-ribose isomerase and that for novel enzymes obtained by the procedure described above. Therefore, the DNAs of the present invention, anti-sense single stranded DNAs and fragments thereof are remarkably useful for the screening of novel enzymes related structurally to L-ribose isomerase.

The following examples explain the present invention in detail:

### Example 1

### DNA encoding L-ribose isomerase

### Example 1-1

### Preparation of chromosomal DNA from Acinetobacter calcoaceticus LR7C

*Acinetobacter calcoaceticus* LR7C (FERM BP-5335) was inoculated into a fresh inorganic salt medium containing D-lyxose as a sole carbon source (D-lyxose inorganic salt medium) and cultivated at 28°C for 24 hours according to the method described by T. Shimonishi in *Journal of Fermentation and Bioengineering,* Vol.81, 493-497 (1996). A seed culture was obtained by repeating the procedure described above three times at every 24 hours. The resulting seed culture was inoculated into 100 ml of a yeast extract medium (pH 7.0) containing 0.5w/v% yeast extract, 0.5w/v% polypeptone, and 0.5w/v% sodium chloride, and cultivated aerobically with agitation and aeration at 28°C overnight. The proliterated microorganisms were harvested from the culture by centrifugation. After conventional lysozyme-treatment, the cells were disrupted by freezing at -80°C and successive dissolution at 60°C. The resulting solution was applied to conventional phenol-extraction, and the resulting water phase was collected and applied to conventional ethanol precipitation. A fraction with a crude chromosomal DNA was obtained as a precipitate and conventionally treated with ribonuclease and proteinase. The resulting chromosomal DNA was subjected to conventional chloroform/isoamylalcohol extraction and then ethanol-precipitation to precipitate the chromosomal DNA. The precipitate was dissolved to give a DNA concentration of 1 mg/ml with sterilized distilled water to obtain a purified preparation of the chromosomal DNA as an aqueous DNA solution.

### Example 1-2

### Preparation of hybridization probe

SEQ ID NO:4 shows the N-terminal amino acid sequence of an L-ribose isomerase, isolated from a microorganism of the genus *Acinetobacter,* as disclosed in Japanese Patent No. 155,480/98 applied for by the same applicant as the present invention. This amino acid sequence has no methionine corresponding to the initial codon at its N-terminus. Therefore, the present inventors hypothesized that the L-ribose isomerase gene of a microorganism of the genus *Aclnetobacter* has a nucleotide sequence encoding an amino acid sequence which has methionine at the N-terminus of SEQ ID NO:4 at the 5'-end of its coding region, and that the L-ribose isomerase is constructed from the polypeptide in which the methionine is deleted from the N-terminus of the preliminal expression product. Based on this hypothesis, sense primers and anti-sense primers for PCR were designed. The nucleotide sequences of the sense primers are shown in SEQ ID NOs:5 and 6. The nucleotide sequences of the anti-sense primers are shown in SEQ ID NOs:7 and 8. The codon, ATG at the 5'-end of SEQ ID NOs:5 and 6 corresponds to methionine as described in the above hypothesis.

The sense and anti-sense primers were chemically synthesized according to the design described above. The PCR was carried out in one system by using the chemically synthesized primers, purified chromosomal DNA prepared in Example 1-1 as a template, and Taq DNA polymerase (Takara Corporation, Tokyo, Japan) under the standard conditions of PCR. As a result, amplification of a DNA having about 70bp chain length was observed. The amplified DNA was cloned into a plasmid vector, pUC119, and sequenced. As a result, the amplified DNA was revealed to be a 68bp DNA encoding the N-terminal amino acid sequence (SEQ ID NO:4), and was judged to be useful for a probe to clone the desired gene. Subsequently, a hybridization probe was prepared by labeling the amplified 68bp DNA with digoxygenin-labeled deoxy-UTP (DIG-11-dUTP) at its 3'-end using commercialized labeling kit (Boehringer Mannheim GmbH, Germany).

### Example 1-3

### Preparation of genomic library of a microorganism of the genus Acinetobacter

About one microgram of the purified chromosomal DNA prepared in Example 1 was partially digested with a restriction endonuclease, *Sac* I, using conventional method. The resulting partial digests were separated by 0.7%(w/v) agarose gel electrophoresis, and transferred to a nylon membrane according to the method of standard southern blotting. The membrane and the probe prepared in Example 1-2 were used for the hybridization. After the hybridization, immunological detection was applied using an anti-DIG antibody. As a result, a remarkable signal showing specific hybridization was detected at a position corresponding to a chain length of 1.2kb. Based on the result, a genomic library of a microorganism of the genus *Acinetobacter* was prepared. Specifically, a 1.2kb fraction was extracted and purified from the gel after the partial digestion of the chromosomal DNA in accordance with the above method and successive agarose gel electrophoresis. The desired genomic library was obtained by inserting the DNA fragments in this fraction to the cloning site of a plasmid vector, pUC119 (ATCC 37461), and successive transformation of *E. coli* DH5 (ATCC 53868).

### Example 1-4

### Screening of genomic library and cloning a DNA encoding L-ribose isomerase

About five thousand clones of the *Acinetobacter* genomic library prepared in Example 1-3 were inoculated into L-agar plate and cultivated at 37°C overnight. The colonies grown on the plate were transferred to and fixed on a nylon membrane. The nylon membrane and the probe prepared in Example 1-2 were used for colony hybridization under standard conditions. After the colony hybridization, immunological detection was applied using an anti-DIG antibody. As a result, it was confirmed that the probe was hybridized on four clones among 5.000 clones screened. These positive clones were isolated and the plasmid DNAs were prepared by conventional method, respectively. The inserted DNAs of each plasmid prepared from four positive clones were sequenced by dideoxy sequencing method. As a result, it was revealed that these nucleotide sequences were completely identical. The nucleotide sequence thus decoded is shown in SEQ ID NO:3. As shown in parallel in SEQ ID NO:3, the sequence encoded an amino acid sequence having methionine at its N-terminus and being constructed by 249 amino acid residues. The amino acid sequence consisting of 2nd to 27th amino acid residues from the N-terminus of an amino acid sequence encoded by the nucleotide sequence of SEQ ID NO:3, was completely identical with the N-terminal amino acid sequence of an L-ribose isomerase isolated from a microorganism of the genus *Acinetobacter.* Based on the result, four positive clones obtained in this example were identified as clones having a DNA which encoded the L-ribose isomerase, i.e., clones having the L-ribose isomerase gene of the microorganism.

The result of sequencing for the above inserted DNA indicates that it should not necessarily require the whole nucleotide sequence of SEQ ID NO:3 to encode an amino acid sequence having L-ribose isomerase activity, and that a nucleotide sequence, which is defective in initial codon and stop codon from the nucleotide sequence, specifically, SEQ ID NO:2 constructed by 744 nucleotides, is sufficient, while an L-ribose isomerase gene from a microorganism of the genus *Acinetobacter* has a nucleotide sequence of SEQ ID NO:3 which is constructed by 750 nucleotides containing the initial codon and the stop codon as a nucleotide sequence for coding region.

The result described in this example indicates that the L-ribose isomerase derived from a microorganism of genus *Acinetobacter* has, generally, the amino acid sequence which is encoded by the nucleotide sequence of SEQ ID NO:2, specifically, the amino acid sequence of SEQ ID NO:1. These results evidence the hypothesis in Example 1-2 by the present inventors.

### Example 2

### Production of polypeptide having L-ribose isomerase activity by recombinant DNA technique

### Example 2-1

### Preparation of recombinant DNA and transformant

A plasmid DNA was isolated from one of positive clones obtained in Experiment 1-4 using conventional method. An inserted DNA having the nucleotide sequence of SEQ ID NO:3 was released from a plasmid DNA by double digestion with two restriction endonucleases, *EcoR* I and *Pst* I. Subsequently, a recombinant DNA, named p8IR, was obtained by the ligation with the released DNA and a plasmid vector, pUC118 (ATCC37462), which had been digested with *EcoR* I and *Pst* I using a ligase. A transformant was obtained by transforming *E. coli* JM109 (ATCC53323) with the recombinant plasmid, p8IR.

### Example 2-2

### Production of polypeptide having L-ribose isomerase activity by transformant

The transformant having the recombinant plasmid, p8IR, was inoculated into 2,000 ml of LB-broth containing 100 mg/L of ampicillin, and cultivated with agitation at 37°C. This cultivation was done while monitoring the turbidity (absorbance at 600 nm, 1 cm cell) of the broth. To the culture was added 5-bromo-4-chloro-3-indolyl-β-galactoside to give a final concentration of 1 mM when the turbidity reached 0.7, and the cultivation was continued for another five hours.

The cells were harvested from the culture broth by centrifugation (10,000×g) for 10 minutes. After the cells were washed two times with glycine-sodium hydroxide buffer (pH 9.0), they were disrupted in the presence of aluminum oxide by using a mortar and pestle. The disrupted cells were suspended in glycine-sodium hydroxide buffer (pH 9.0), and the cell-debris were removed from the suspension by centrifugation (10,000×g) for 30 minutes. The desired activity was detected when the resulting crude extract was applied to the assay of L-ribose isomerase activity. The activity was calculated to be about 1.5 U/ml-broth. No L-ribose isomerase activity was detected when *E. coli* JM109 having the plasmid vector pUC118 was substituted for the recombinant plasmid, p8IR, then cultured and disrupted similarly as above. This result supports that the DNA, cloned and sequenced in Experiment 1, encodes L-ribose isomerase.

### Example 3

### Preparation of polypeptide having L-ribose isomerase activity

According to the method described in Japanese Patent Kokai No. 155,480/98 by the same applicant as the present invention, a polypeptide having L-ribose isomerase activity was purified by successive polyethylene fractionation, anion-exchange chromatography and gel filtration chromatography from the crude cell extract which was obtained in Example 2-3.

The purified polypeptide showed a single protein band at a position corresponding to the molecular weight of 28,000 daltons by conventional SDS-PAGE. This result well agreed with the data that the molecular weight of L-ribose isomerase from a microorganism of the genus *Acinetobacter* is in the range between about 25,000 and about 35,000 daltons, which is disclosed in Japanese Patent Kokai No. 155,480/98 by the same applicant as the present invention.

The enzymatic properties of the purified polypeptide described above were investigated using L-ribose isomerase activity according to the assay. The optimum temperature was about 30°C under the reaction conditions of at pH 9.0 for 10 minutes. The thermal stability was about 30°C or lower under the 10 minutes-incubation at pH 9. The optimum pH was in the range of pH of about 8 to about 9. The pH stability was in the range of pH of about 7 to about 9 under the 24 hours-incubation at 4°C. D-Lyxose and D-mannose were converted by the action of the purified polypeptide into D-xylulose and D-fructose, respectively, when D-lyxose and D-mannose were used as the substrates for the polypeptide. This result indicates that the polypeptide in this Example catalyzes the isomerization reaction of D-ribose, as well as D-lyxose and D-mannose. These results also well agree with the enzymatic properties of L-ribose isomerase from a microorganism of the genus *Acinetobacter* as disclosed in Japanese Patent Kokai No.155,480/98 by the same applicant as the present invention.

These results evidence that the polypeptide obtained by using the DNA of the present invention can be advantageously useful for the industrial production of various rare sugars similarly as in the case of L-ribose isomerase which is originally produced by naturally-occurring microorganisms.

As is evident from the above, the present invention was made based on the cloning of a novel DNA encoding an L-ribose isomerase by the present inventors. The DNA of the present invention enables to supply useful enzymes for the industrial production of rare sugars such as L-ribose and D-talose by the recombinant DNA technique at a lesser cost and more constant quality than conventional methods. It is possible that novel enzymes can be obtained by screening chromosomes from cells including various microorganisms using the DNA of the present invention or fragments thereof as hybridization probes or PCR-primers. Thus, the DNA of the present invention and fragments thereof are specifically useful for the screening of novel saccharide-related enzymes.

The present invention is a significant invention that contributes to various fields such as foods and pharmaceuticals.

While there has been described what is at present considered to be the preferred embodiments of the invention, it will be understood the various modifications may be made therein, and it is intended to cover in the appended claims all such modifications as fall within the true spirit and scope of the invention.

## Claims

1. A DNA encoding an L-ribose isomerase which isomerizes L-ribose into L-ribulose and *vice versa.*

2. The DNA of claim 1, wherein said L-ribose isomerase comprises a part or the whole of the amino acid sequence of SEQ ID NO:1.

3. The DNA of claim 1 or 2, which comprises a part or the whole of the nucleotide sequence of SEQ ID NO:2.

4. The DNA of claim 1, 2, or 3, which is obtainable from a microorganism of the genus *Acinetobacter.*

5. A DNA which is a member selected from the group consisting of DNAs as fragments of the DNA of any one of claims 1 to 4, single-stranded DNAs as anti-sense DNAs which correspond to the sense strand of the DNA of any one of claims 1 to 4, and fragments of the single-stranded DNAs.

6. A hybridization probe or PCR-primer which comprises the DNA of claim 5.

7. A recombinant DNA, which comprises the DNA of any one of claims 1 to 4.

8. A transformant which is constructed by transforming a host-cell using either the DNA of any one of claims 1 to 4 or a recombinant DNA comprising the DNA of any one of claims 1 to 4.

9. The transformant of claim 8, wherein said host-cell is a microorganism.

10. A process for producing a polypeptide, which comprises the steps of artificially expressing the DNA of any one of claims 1 to 4 to form a polypeptide having an activity to isomerize L-ribose into L-ribulose and *vice versa,* and collecting the formed polypeptide.

11. The process of claim 10, which comprises the steps of culturing a transformant constructed by transforming the DNA of any one of claims 1 to 4, and artificially allowing to express the DNA of any one of claims 1 to 4.

12. The process of claim 10 or 11, wherein the formed polypeptide is collected by one or more techniques selected from the group consisting of dialysis, salting out, filtration, concentration, separatory precipitation, gel filtration chromatography, ion-exchange chromatography, hydrophobic chromatography, reverse-phase chromatography, affinity chromatography, gel electrophoresis, and isoelectric focusing.

13. A polypeptide which is obtainable by the process of any one of claims 10 to 12.


SEQUENCE LISTING

<110> Kabushiki Kaisha Hayashibara Seibutsu Kagaku Kenkyujo

<120> DNA encoding L-ribose isomerase and uses thereof

<130> 8

<160> 10088701

<210> 1
<211> 248
<212> PRT
<213> Acinetobacter sp.

<400> 1
Thr Arg Thr Ser Ile Thr Arg Arg Glu Tyr Asp Glu Trp Val Arg Glu
1               5                   10                  15
Ala Ala Ala Leu Gly Lys Ala Leu Arg Tyr Pro Ile Thr Glu Lys Met
            20                  25                  30
Val Asn Asp Ser Ala Arg Ile Val Phe Gly Ala Asp Gln Tyr Asp Ala
        35                  40                  45
Phe Lys Asn Gly Met Trp Ser Gly Glu Pro Tyr Glu Ala Met Ile Ile
    50                  55                  60
Phe Glu Ser Leu Asn Glu Pro Ala Val Asp Gly Leu Pro Thr Gly Ala
65                  70                  75                  80
Ala Pro Tyr Ala Glu Tyr Ser Gly Leu Cys Asp Lys Leu Met Ile Val
                85                  90                  95
His Pro Gly Lys Phe Cys Pro Pro His His His Gly Arg Lys Thr Glu
            100                 105                 110
Ser Tyr Glu Val Val Leu Gly Glu Met Glu Val Phe Tyr Ser Pro Thr
        115                 120                 125
Pro Ser Ala Glu Ser Gly Val Glu Leu Leu Asn Phe Ser Gly Met Pro
    130                 135                 140
Val Gly Ser Pro Trp Pro Glu Gly Val Ala Leu Pro Lys Gly Arg Glu
145                 150                 155                 160
Ser Ser Tyr Glu Lys Leu Thr Ser Tyr Val Arg Leu Arg Ala Gly Asp

                    165                     170                     175
Pro Lys Phe Val Met His Arg Lys His Leu His Ala Phe Arg Cys Pro
                180                     185                     190
Pro Asp Ser Asp Val Pro Leu Val Val Arg Glu Val Ser Thr Tyr Ser
            195                     200                     205
His Glu Pro Thr Glu Ala Ala Ala Gly Asn His Ala Pro Ile Pro Ser
        210                     215                     220
Trp Leu Gly Met His Asp Asn Asp Phe Val Ser Asp Ala Ala Asn Thr
225                     230                     235                     240
Gly Arg Leu Gln Thr Ala Ile Ser
                    245

<210> 2
<211> 744
<212> DNA
<213> Acinetobacter sp.

<400> 2
aca agg acg tcg att act cgt cgc gag tat gac gaa tgg gtg cgg gag      48
Thr Arg Thr Ser Ile Thr Arg Arg Glu Tyr Asp Glu Trp Val Arg Glu
  1               5                  10                  15
gct gcc gcc ctc ggc aaa gcc ctt cgt tac ccg att acc gaa aaa atg      96
Ala Ala Ala Leu Gly Lys Ala Leu Arg Tyr Pro Ile Thr Glu Lys Met
                 20                  25                  30
gtc aac gac tca gct cgc atc gtc ttc ggc gcc gac cag tat gac gcc     144
Val Asn Asp Ser Ala Arg Ile Val Phe Gly Ala Asp Gln Tyr Asp Ala
             35                  40                  45
ttc aag aat ggc atg tgg tcg ggt gag ccg tac gaa gcg atg atc atc     192
Phe Lys Asn Gly Met Trp Ser Gly Glu Pro Tyr Glu Ala Met Ile Ile
         50                  55                  60
ttc gaa tcg ctg aac gag ccc gcc gtc gac ggc ctg ccg aca ggc gcg     240
Phe Glu Ser Leu Asn Glu Pro Ala Val Asp Gly Leu Pro Thr Gly Ala
 65                  70                  75                  80
gca ccc tat gcg gag tac tcg ggc ctg tgc gac aag ctg atg atc gtc     288
Ala Pro Tyr Ala Glu Tyr Ser Gly Leu Cys Asp Lys Leu Met Ile Val
                 85                  90                  95
cac ccg ggg aag ttt tgc cca cca cat cac cac ggg aga aag acc gag     336

His Pro Gly Lys Phe Cys Pro Pro His His His Gly Arg Lys Thr Glu
            100                 105                 110
agc tat gag gtc gtc ctt ggc gag atg gaa gtc ttc tac agt ccg act 384
Ser Tyr Glu Val Val Leu Gly Glu Met Glu Val Phe Tyr Ser Pro Thr
        115                 120                 125
ccg tca gcg gag tcc ggc gtc gag ttg ctg aac ttc tcg ggc atg cca 432
Pro Ser Ala Glu Ser Gly Val Glu Leu Leu Asn Phe Ser Gly Met Pro
    130                 135                 140
gtt ggg agc ccg tgg ccc gag ggg gtc gcg ttg ccc aag ggg cgc gag 480
Val Gly Ser Pro Trp Pro Glu Gly Val Ala Leu Pro Lys Gly Arg Glu
145                 150                 155                 160
agt tca tac gaa aag cta acc agc tac gtg cga cta cgc gcc ggt gac 528
Ser Ser Tyr Glu Lys Leu Thr Ser Tyr Val Arg Leu Arg Ala Gly Asp
                165                 170                 175
ccg aag ttc gtc atg cac cgc aag cac ttg cac gct ttc cgc tgc ccc 576
Pro Lys Phe Val Met His Arg Lys His Leu His Ala Phe Arg Cys Pro
            180                 185                 190
ccc gac tcg gac gtt ccg ttg gtc gtc cga gaa gtc tca acc tac agc 624
Pro Asp Ser Asp Val Pro Leu Val Val Arg Glu Val Ser Thr Tyr Ser
        195                 200                 205
cac gag ccc act gaa gct gcc gcc ggc aac cat gct ccg atc cct agc 672
His Glu Pro Thr Glu Ala Ala Ala Gly Asn His Ala Pro Ile Pro Ser
    210                 215                 220
tgg ctg ggc atg cac gac aac gat ttt gtc tcg gac gct gcc aac acc 720
Trp Leu Gly Met His Asp Asn Asp Phe Val Ser Asp Ala Ala Asn Thr
225                 230                 235                 240
ggg cgg ctt cag acc gcg atc agc 744
Gly Arg Leu Gln Thr Ala Ile Ser
                245

<210> 3
<211> 750
<212> DNA
<213> Acinetobacter sp.

<220>
<221> CDS

<222> (1)...(750)

<220>
<221> mat_protein
<222> (4)...(747)

<400> 3
atg aca agg acg tcg att act cgt cgc gag tat gac gaa tgg gtg cgg     48
Met Thr Arg Thr Ser Ile Thr Arg Arg Glu Tyr Asp Glu Trp Val Arg
 1               5                  10                  15
gag gct gcc gcc ctc ggc aaa gcc ctt cgt tac ccg att acc gaa aaa     96
Glu Ala Ala Ala Leu Gly Lys Ala Leu Arg Tyr Pro Ile Thr Glu Lys
            20                  25                  30
atg gtc aac gac tca gct cgc atc gtc ttc ggc gcc gac cag tat gac    144
Met Val Asn Asp Ser Ala Arg Ile Val Phe Gly Ala Asp Gln Tyr Asp
        35                  40                  45
gcc ttc aag aat ggc atg tgg tcg ggt gag ccg tac gaa gcg atg atc    192
Ala Phe Lys Asn Gly Met Trp Ser Gly Glu Pro Tyr Glu Ala Met Ile
    50                  55                  60
atc ttc gaa tcg ctg aac gag ccc gcc gtc gac ggc ctg ccg aca ggc    240
Ile Phe Glu Ser Leu Asn Glu Pro Ala Val Asp Gly Leu Pro Thr Gly
65                  70                  75                  80
gcg gca ccc tat gcg gag tac tcg ggc ctg tgc gac aag ctg atg atc    288
Ala Ala Pro Tyr Ala Glu Tyr Ser Gly Leu Cys Asp Lys Leu Met Ile
                85                  90                  95
gtc cac ccg ggg aag ttt tgc cca cca cat cac cac ggg aga aag acc    336
Val His Pro Gly Lys Phe Cys Pro Pro His His His Gly Arg Lys Thr
            100                 105                 110
gag agc tat gag gtc gtc ctt ggc gag atg gaa gtc ttc tac agt ccg    384
Glu Ser Tyr Glu Val Val Leu Gly Glu Met Glu Val Phe Tyr Ser Pro
        115                 120                 125
act ccg tca gcg gag tcc ggc gtc gag ttg ctg aac ttc tcg ggc atg    432
Thr Pro Ser Ala Glu Ser Gly Val Glu Leu Leu Asn Phe Ser Gly Met
    130                 135                 140
cca gtt ggg agc ccg tgg ccc gag ggg gtc gcg ttg ccc aag ggg cgc    480
Pro Val Gly Ser Pro Trp Pro Glu Gly Val Ala Leu Pro Lys Gly Arg
145                 150                 155                 160

gag agt tca tac gaa aag cta acc agc tac gtg cga cta cgc gcc ggt 528
Glu Ser Ser Tyr Glu Lys Leu Thr Ser Tyr Val Arg Leu Arg Ala Gly
                165                 170                 175
gac ccg aag ttc gtc atg cac cgc aag cac ttg cac gct ttc cgc tgc 576
Asp Pro Lys Phe Val Met His Arg Lys His Leu His Ala Phe Arg Cys
            180                 185                 190
ccc ccc gac tcg gac gtt ccg ttg gtc gtc cga gaa gtc tca acc tac 624
Pro Pro Asp Ser Asp Val Pro Leu Val Val Arg Glu Val Ser Thr Tyr
        195                 200                 205
agc cac gag ccc act gaa gct gcc gcc ggc aac cat gct ccg atc cct 672
Ser His Glu Pro Thr Glu Ala Ala Ala Gly Asn His Ala Pro Ile Pro
    210                 215                 220
agc tgg ctg ggc atg cac gac aac gat ttt gtc tcg gac gct gcc aac 720
Ser Trp Leu Gly Met His Asp Asn Asp Phe Val Ser Asp Ala Ala Asn
225                 230                 235                 240
acc ggg cgg ctt cag acc gcg atc agc tag 750
Thr Gly Arg Leu Gln Thr Ala Ile Ser
                245

<210> 4
<211> 26
<212> PRT
<213> Acinetobacter sp.

<400> 4
Thr Arg Thr Ser Ile Thr Arg Arg Glu Tyr Asp Glu Trp Val Arg Glu
1               5                   10                  15
Ala Ala Ala Leu Gly Lys Ala Leu Arg Tyr
            20                  25

<210> 5
<211>
<212> DNA
<213> Artificial Sequence

<220>
<223> Oligonucleotide as PCR sense primer designed from N-terminal

amino acid sequence of Acinetobacter sp. L-ribose isomerase

<400> 5
atgacimgia cnwsiathac                                                    20

<210> 6
<211>
<212> DNA
<213> Artificial Sequence

<220>
<223> Oligonucleotide as PCR sense primer designed from N-terminal
amino acid sequence of Acinetobacter sp. L-ribose isomerase

<400> 6
atgacsmgma cnwsnathac                                                    20

<210> 7
<211>
<212> DNA
<213> Artificial Sequence

<220>
<223> Oligonucleotide as PCR antisense primer designed from
N-terminal amino acid sequence of Acinetobacacter sp. L-ribose
isomerase

<400> 7
ytcigtdati ggrtaickna                                                    20

<210> 8
<211>
<212> DNA
<213> Artificial Sequence

<220>
<223> Oligonucleotide as PCR antisense primer designed from

N-terminal amino acid sequence of Acinetobacacter sp. L-ribose
isomerase

<400> 8
ttnccnarng cngcngyctc                                                    20